# EUROPEAN PATENT SPECIFICATION

(11) **EP 2 157 943 B1**
(45) Date of publication and mention of the grant of the patent: **25.06.2014**
(21) Application number: 07853063.1
(22) Date of filing: 30.10.2007
(51) Int. Cl.: A61F 2/88, A61M 29/00, A61M 25/00, A61F 2/95, A61M 29/02, A61F 2/958

(54) **An assembly with non-occluding dilation device**
Aufbau mit nicht-okkludierende Dilatationsvorrichtung
Ensemble aved dispositif de dilatation non-occlusif

(30) Priority: 19.06.2007 US 820726; 23.10.2007 US 977415
(43) Date of publication of application: 03.03.2010
(73) Proprietor: Lemaitre Vascular, Inc., Burlington, MA 01803 (US)
(72) Inventor: RAMAIAH, Venkatesh, Scottsdale, AZ 85259 (US); MCNUTT, Robert, Mesa, AZ 85206 (US)
(74) Representative: Cordina, Kevin John
(86) International application number: PCT/US2007/023087
(87) International publication number: WO 2008/156468

(56) References cited:
- EP-A- 1 574 169
- WO-A-91/01773
- WO-A-96/17645
- WO-A-97/46174
- WO-A-2007/002863
- WO-A-2008/005405
- WO-A1-01/37921
- WO-A2-2007/089897
- FR-A- 2 735 967
- US-A- 3 517 128
- US-A- 4 921 484
- US-A- 5 180 368
- US-A- 5 257 975
- US-A- 5 354 310
- US-A- 5 626 602
- US-A- 5 817 102
- US-A- 5 954 742
- US-B1- 6 312 407
- US-B1- 6 497 722
- US-B1- 6 635 068

## Description

### Field of the Invention

The present invention relates generally to medical devices, and more particularly to a medical device for the dilation of blood vessels and/or the dilation of structures positioned within blood vessels.

### Background of the Invention

Conventional systems for dilating blood vessels and/or structures (e.g., stents or stent grafts) positioned in a blood vessel utilize balloon-like structures ("balloon dilators"). Such structures are typically made from essentially impermeable materials. When such a device is expanded to perform a dilation, blood flow is entirely or substantially occluded through the blood vessel in which the balloon dilator is being used. Such an occlusion of blood flow could, if continued for too long, harm the patient, since portions of the body downstream of the balloon dilator will not receive blood while the flow is occluded or substantially hindered. Thus, the length of time balloon dilators may be dilated is limited and this can hinder proper completion of the dilation procedure.

A similar problem with balloon dilators arises when a dilation procedure is being performed in a portion of the circulatory system where there is a branch in the blood vessels, such as where the Illac or renal arteries are side vessels that branch from the aorta. In that case a balloon dilator may cover a side vessel and partially or totally occlude blood flow to the side vessel.

Another problem with balloon-like dilators is called the "windsock effect." Because blood flow is substantially or entirely occluded when balloon dilators are dilated, the blood pressure upstream of the balloon dilator can be significant and may cause the balloon dilator, and any structure (such as a stent or stent graft) positioned in the blood vessel and that is being dilated by the balloon dilator, to move out of the desired position, effectively pushed down stream (i.e., in the antegrade direction) by the upstream blood pressure. Because of this problem accurate placement of such structures can be difficult utilizing balloon dilators

WO96/17645 discloses a vascular dilation device for enlarging a passage, comprising a cylindrical dilation member with a flow passage with a diameter and a longitudinally extending axis extending there through.

WO2007/002863 discloses a device for dilating either a vessel within a body or a structure positioned within the vessel and is designed so that it does not occlude or substantially hinder the flow of blood through the vessel.

EP 1574169 discloses an occluding system, the system comprising a plurality of shape memory wires woven together to form a body useful for occluding an anatomical structure.

WO 01/37921 discloses a cannula with a flexible shaft composed of a tubular body and expandable scaffolding useful for standard gravity drainage or vacuum assisted/suction drainage.

WO 2007/089897 discloses a method for increasing blood flow through an obstructed blood vessel by providing an expandable member made of a mesh having a plurality of interstices.

### Definitions

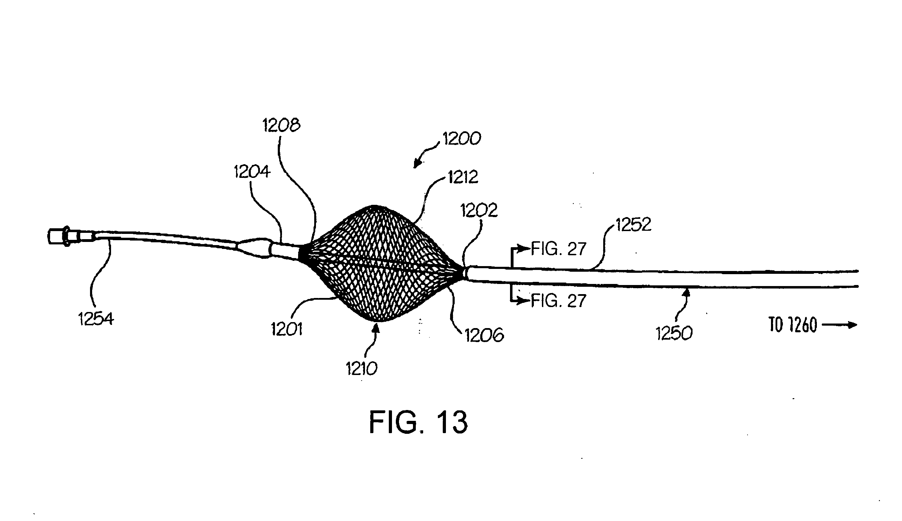

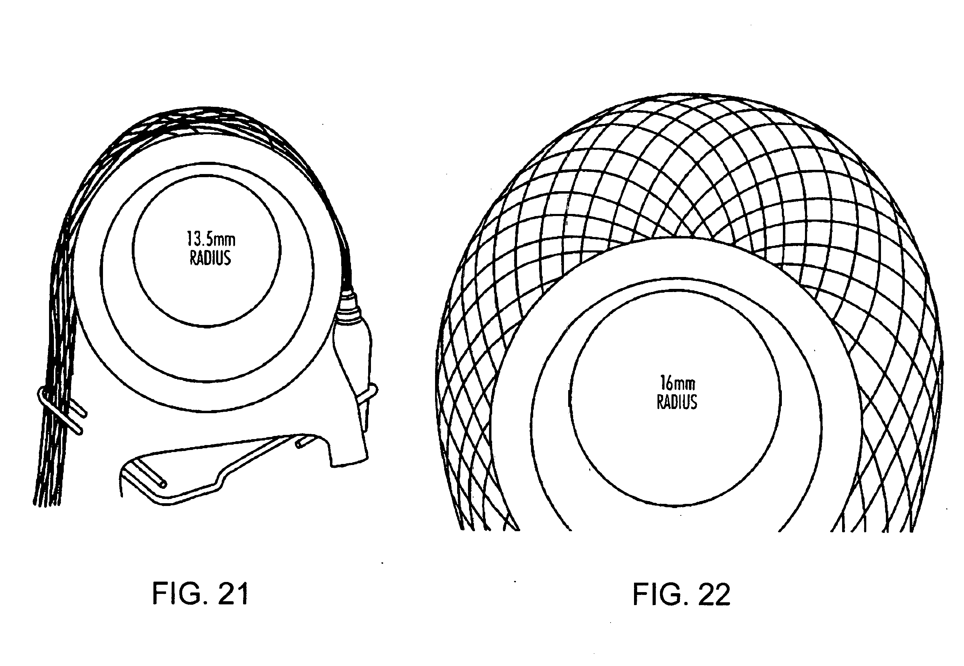

As used herein, in addition to the other terms defined in this disclosure, the following terms shall have the following meanings:
"Assembly" means a device according to the invention assembled as part of or connected to a catheter so that it can be advanced into a vessel.
"Collapsed" when referring to a device according to the invention means that the device is in its relaxed, undilated position. The device would normally be in its collapsed position when introduced into a vessel and/or when retained within a cover sheath of a triaxial catheter.
"Contraction" of a device or "contracting" a device means that its diameter is being or has been reduced from a dilated position.
"Criss-cross" pattern means a wire pattern wherein the wires cross one another as shown, for example, in Figures 13 - 20.
"Device" or "dilation device" means a structure for (a) dilating a vessel, and/or (b) dilating a structure inside of one or more vessels (such as an endograft stent or stent graft) to be deployed or repositioned within one or more vessels.
"Diameter" as used in connection with a vessel means the approximate diameter of a vessel since vessels are seldom perfectly cylindrical. "Diameter" as used with respect to any man-made structure means the approximate diameter.
"Diameter disparity ratio" means the disparity of the diameter of a single vessel. Vessels, particularly diseased vessels, may not have a relatively constant diameter and the diameter can suddenly increase or decrease. For example, the diameter of a vessel may suddenly change from an initial diameter to a diameter of 1.5 times the initial diameter, in which case the diameter disparity ratio would be 1.5:1. A diameter disparity ratio or multi-vessel diameter disparity ratio (as defined below) to which a device according to some aspects of the invention could conform is one or more of the ratios between 1.2:1 and 3.4:1, including diameter disparity ratios of 1.2:1, 1.4:1, 1.6:1, 1.8:1, 2.0:1, 2.2:1, 2.4:1, 2.6:1 and 2.8:1, 3.0:1 and 3.4:1.
"Dilated" refers to a device according to the invention when it is expanded. A device dilated within a vessel may be dilated for the purpose of dilating the vessel itself and/or for dilating a structure within the vessel. "Expanded" and "dilated" have the same meaning when used in connection with a device according to the invention.
"Fluid" means any bodily fluid, such as blood.
"Fully dilated" or "fully expanded" means the maximum amount a device according to the invention can be dilated (as measured at its greatest diameter) when unhindered by external structures (such as a vessel) and when dilated using the delivery system of a catheter to which the device is attached.
"Kink radius" refers to the radius to which a device according to the invention can be formed without the device permanently deforming (i.e., without "kinking"). If the device is mounted on a catheter the kink radius refers to the kink radius of the entire assembly, i.e., the device mounted to a biaxial or triaxial catheter (with the sheath covering the device), since the entire assembly moves through the vessel when the device is advanced into place. The lower the kink radius the greater the resistance of the device or assembly to kinking. Figures 21-23 show measurement of the kink radius with respect to an embodiment of the present invention. "Kink radius" and methods for testing same are discussed in "Pigtail Catheters Used for Percutaneous Fluid Drainage: Comparison of Performance Characteristics," Douglas B. Macha, John Thomas and Rendon C. Nelson, Radiology vol. 238: Number 3 (March 2006).
"Multi-vessel diameter disparity ratio" means the disparity of the diameters of two vessels. When a device according to the invention is used it may be deployed and dilated within two vessels simultaneously and the two vessels may have different, respective diameters. For example, if one vessel has a first diameter and the second vessel has a second diameter 1.8 times as large as the first diameter, the multi-vessel diameter disparity ratio would be 1.8: 1. A device according to some aspects of the invention could conform to one or more of the multi-vessel diameter ratios between 1.2: 1 and 3.4: 1.
"Pressure drop" means the reduction in pressure in part of a vessel when a device is (a) dilated within the vessel, or (b) dilated in another vessel but totally or partially covering the opening to the vessel (in which case the vessel may be referred to as a "side vessel"). When a balloon dilator is fully dilated within a vessel the pressure upstream of the balloon dilator increases significantly while the pressure downstream of the balloon dilator, or in a side vessel covered by the balloon dilator, can reach substantially zero (meaning that the balloon dilator has blocked most or all of the blood flow). As an example, if the pressure at a location in a vessel is 100 mm Hg (i.e., a pressure of 100 millimeters of mercury) before a device is dilated within the vessel, and the pressure at the same location in the vessel is 10 mm HG after the device is dilated, the pressure drop would be 90%, i.e., 100 - 10 = 90, and 90/100 = 90%. Similarly, for the same vessel if the pressure after dilation were 20 mm Hg the pressure drop would be 80%, if the pressure after dilation were 30 mm Hg the pressure drop would be 70%, if the pressure after dilation were 5 mm Hg the pressure drop would be 95% and if the pressure after dilation were 1 mm Hg the pressure drop would be 99%.
"Strut" means a wire having a generally rectangular (preferably with radiused edges) cross-section with generally flat top and bottom surfaces and having a width greater than its thickness.
"Vessel" means any vessel within a body, such as the human body, through which blood or other fluid flows and includes arteries and veins.
"Vessel flow path" means the direction of fluid flow through a vessel.
"Wire" means any type of wire, strand, strut or structure, regardless of cross-sectional dimension (e.g., the cross-section could be circular, oval, or rectangular) or shape, and regardless of material, that may be used to construct a device as described or claimed herein. Some wires may be suitable for one or more of the embodiments but not suitable for others.

### Summary of the Invention

The present invention provides an assembly comprising a dilation device for dilating a vessel or a structure positioned within a vessel, the device comprising a plurality of wires comprised of nitinol and having a generally circular cross section and a diameter of between 0.203 mm (0.008 inches) and 0.457 mm (0.018 inches), wherein the plurality of wires form a body portion having a proximal end and a distal end, wherein the plurality of wires form a criss-cross pattern and are spaced apart so that at least some of the spaces between the wires has an area of between 1 mm² and 400 mm² when the device is dilated to allow for passage of fluid through the device, the device being sufficiently compliant to conform to at least one diameter disparity ratio of between about 1.2:1 and about 3.4:1 when dilated, wherein the diameter disparity ratio is the disparity of the diameter of the vessel, the assembly further comprising a catheter for engaging the device, wherein a first retention end of the catheter engages the proximal end of the body portion and a second retention end of the catheter engages the distal end of the body portion.

A selection of optional features is set out in the dependent claims.

The descriptions of the invention herein are exemplary only and are not restrictive of the invention as claimed.

### Brief Description of the Drawings

Figures 1A-E show examples of dilation devices according to various aspects of the invention.
Figures 2A-C show a spiraled dilation device according to one embodiment of the invention.
Figures 3A-D show additional views of a spiraled dilation device according to one embodiment of the invention.
Figures 4A-C show a non-spiraled, dilation device according to one embodiment of the invention.
Figures 5A-B show another non-spiraled, dilation device according to one embodiment of the invention.
Figures 6A-B show a delivery and deployment system for a non-spiraled, dilation device according to one embodiment of the invention.
Figure 7 shows a control mechanism for a dilation device according to one embodiment of the invention.
Figure 8 is a side view of an alternate device according to the invention in a dilated position and showing a band 808 in its body.
Figure 9 shows a side view of an alternate device according to the invention in a dilated position and having wires that are parallel to the vessel flow path when the device is positioned in a vessel.
Figure 10 shows a side view of an alternate device according to the invention in a dilated position and having wires that are parallel to the vessel flow path when the device is positioned in a vessel.
Figure 11 shows a side view of an alternate device according to the invention in a dilated position and having wires that are parallel to the vessel flow path when the device is positioned in a vessel.
Figure 12 is another side view of the device of Figure 11.
Figure 13 is a side view of an alternate embodiment of a device according to the invention and mounted on a catheter, wherein the device comprises wires formed in a criss-cross pattern.
Figure 14 is a close up, partial side view of the device shown in Figure 13.
Figure 15 is another view of the device and catheter shown in Figure 13 illustrating how the device can be used to dilate a stent graft.
Figure 16 is a partial, side view of an alternate device according to the invention simulating how the device conforms to a diameter disparity ratio within a vessel.
Figure 17 is a view of the device and catheter of Figure 13 simulating how the device conforms to a multi-vessel diameter disparity ratio.
Figure 18 is a view of the device of Figure 16 simulating how the device conforms to a multi-vessel diameter disparity ratio and simultaneously conforms to an asymmetrical vessel shape.
Figure 19 is another view of the device of Figure 16 simulating how the device conforms to a diameter disparity ratio within a vessel and showing the device covering side vessels.
Figure 20 is another view of the device of Figure 13 simulating the device placed in the aorta and covering the renal arteries.
Figure 21 is another view of the device of Figure 13 showing that it has a kink radius of at least 13.5 mm when collapsed.
Figure 22 is another view of the device of Figure 13 showing that it has a kink radius of at least 16 mm when fully dilated.
Figure 23 is another view of the device of Figure 13 showing that it has a kink radius of at least 20 mm when fully dilated.
Figure 24 is a side, perspective view of the catheter and device of Figure 13.
Figure 25 is a top view of the proximal end of the catheter of Figure 13 with the device enclosed within the catheter's outer sheath.
Figure 26 is a top view of the proximal end of the catheter of Figure 13.
Figure 27 is a cross-sectional depiction of a triaxial catheter that can be used to move a device into a vessel.

### Detailed Description of Preferred Embodiments

A device according to the invention is for dilating a vessel and/or a structure (such as an endograft, stent or stent graft) positioned in the vessel, or alternatively may be used to simultaneously dilate two vessels or dilate a structure positioned in two vessels. The device comprises a plurality of wires and has a first position wherein it is collapsed. In this first position the device has a sufficiently small enough diameter to be positioned in a vessel where it is to be used. The device also has a second position wherein it is dilated in order to dilate a vessel and/or a structure within the vessel. When dilated the wires of the device are spaced apart to allow for the passage of fluid through the device. Thus, the device is designed so that it does not occlude or substantially hinder the flow of fluid through the vessel.

A device according to the invention may have a collapsed diameter sufficient to fit into any suitable catheter. A device according to the invention may fit into a 12 french diameter catheter, a 15 french diameter catheter, or any other catheter suitably sized for a procedure utilizing the device. The fully expanded diameter of a device according to the invention is preferably between 30 mm and 55 mm. In one embodiment the collapsed diameter is slightly less than 12 french and the fully expanded diameter is between 30 mm and 35 mm. In another embodiment the collapsed diameter is slightly less than 15 french and the fully expanded diameter is between 50 mm and 55 mm.

A device according to the invention may also be configured to have a fully expanded diameter of 15% greater than the diameter of a vessel at the location in the vessel at which the device is to be dilated.

A device according to the invention may also have any suitable length, such as any length of between 4 cm (centimeters) and 15 cm between the distal end and the proximal end when the device is in its fully collapsed position. Some preferred lengths are between 4 cm and 15 cm, between 6 cm and 15 cm, between 8 cm and 15 cm, between 10 cm and 15 cm, and between 12 cm and 15 cm.

A device according to the invention exerts a radial force when being dilated, wherein the radial force is sufficient to dilate a stent or stent graft with which the device is used. The radial pressure can be between 34.5 kpa (5 pounds per square inch (psi)) and 137.9 kpa (20 psi), between 41.4 kpa (6 psi) and 137.9 kpa (20 psi), between 48.3 kpa (7 psi) and 137.9 kkpa (20 psi), between 8 psi and 20 psi, between 9 psi and 20 psi, between 10 psi and 137.9 kpa (20 psi) or between 103.4 kpa (15 psi) and 137.9 kpa (20 psi). The radial pressure may vary within a given range depending upon the diameter of the device (e.g., the radial pressure may decrease as the diameter of the device increases). The radial pressure within a given, suitable psi range is preferably exerted over the entire working range of the device. The "working range" means all diameters of the device at which the device is expanding a stent or stent graft. In one embodiment, the measured radial force exerted at given diameters was 64.8 kpa (9.4 psi) at a diameter of 20 mm, 46.2 kpa (6.7 psi) at a diameter of 30 mm and 43.4 kpa (6.3 psi) at a diameter of 40 mm. A device according to the invention preferably exerts a radial pressure of between 34.5 kpa (5 psi) and 137.9 kpa (20 psi) over at least part, and preferably over all, of its working range.

Some devices according to the invention are also sufficiently compliant (or flexible) so that when placed in a vessel and dilated they conform to the dimensions of the vessel even when the vessel dimensions are not uniform. In particular, some devices of the present invention can conform to one or more diameter disparity ratios of between 1.2: 1 and 3.4: 1 and some devices according to the invention can conform to one or more multi-vessel diameter disparity ratios of between 1.2: 1 and 3.4: 1.

The wires used in a device according to the invention may be of any suitable size, shape, thickness and material. For example, all or some of the wires may have a generally circular cross-section and have a diameter of between 0.2mm (.008") and 0.46mm (.018"). Alternatively, all or some of the wires may include one or more struts that have a thickness of between 0.2mm (.008") and 4.6mm (.018") and a width of between 0.5mm (.020") and 1.27mm (.050"). A wire may be comprised of stainless steel, nitinol, cobalt, chromium or any suitable metal, plastic or other suitable material. In one preferred embodiment, the wire is comprised of nitinol, has a generally circular cross section and a diameter of about 0.4mm (.015"). In this embodiment, the wires are formed in a criss-cross pattern (as shown in Figures 13- 20).

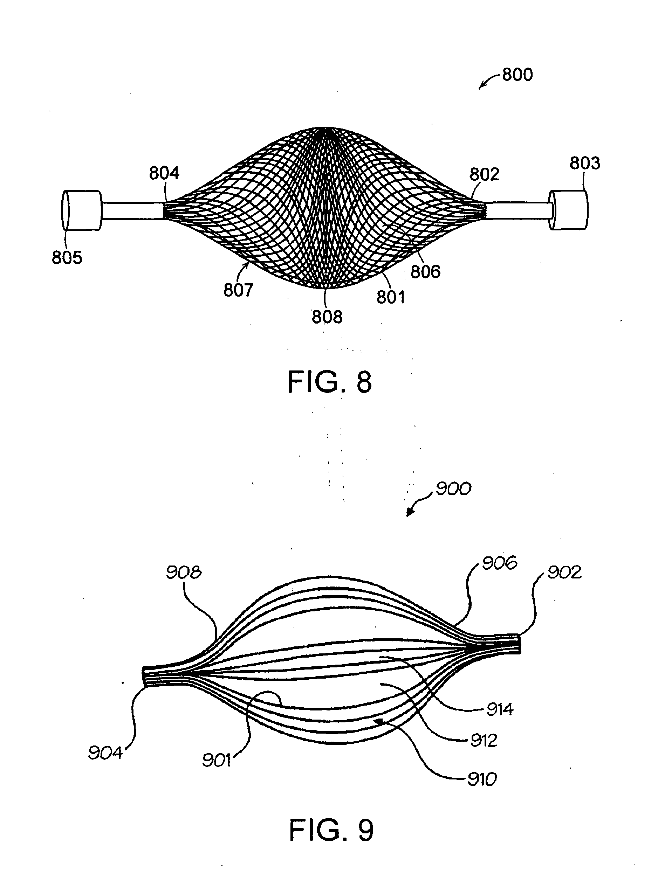

The device may have any suitable density of wires and the wires may be formed in any suitable pattern, such as in a criss-cross pattern or in a non-overlapping pattern in which the wires are parallel to vessel flow path (as shown in Figures 9-12).

If a device according to the invention has wires that are parallel (as used in this context, "parallel" means the wires are substantially parallel to one another) to the vessel flow path, the device may have between four and twenty-four wires, or may have more than twenty-four wires. In various embodiments, a device according to the invention includes, respectively, four wires, five wires, six wires, seven wires, eight wires, nine wires, ten wires, eleven wires, twelve wires, thirteen wires, fourteen wires, fifteen wires, sixteen wires, seventeen wires, eighteen wires, nineteen wires, twenty wires, twenty-one wires, twenty-two wires, twenty-three wires and twenty-four wires. The maximum distance between each wire in such a device can vary depending upon the number of wires, the width of the wires and the proposed use of the device, but generally the maximum distance between wires will be between 1 mm and 100 mm when the device is fully dilated. In various embodiments of the device, the maximum distance is, respectively, no greater than 1 mm, 5 mm, 10 mm, 15 mm, 20 mm, 25 mm, 30 mm, 35 mm, 40 mm, 45 mm, 50 mm, 55 mm, 60 mm, 65 mm, 70 mm, 75 mm, 80 mm, 85 mm, 90 mm, 95 mm or 100 mm.

If a device according to the invention includes wires in a criss-cross pattern, each of the largest spaces between the wires when the device is fully dilated could have an area of between 1 mm² and 400 mm², including areas of 1 mm², 2 mm², 4 mm², 10 mm², 25 mm², 50 mm², 75 mm², 100 mm², 150 mm², 200 mm², 250 mm², 300 mm², 350 mm², and/or 400 mm² or areas within that range. It is also possible that the area of the largest spaces could be larger than 400 mm² or smaller than 1 mm², as long as the device falls within the scope of one of the claims and works for its intended purpose of dilating a vessel or dilating a structure within a vessel without occluding or substantially hindering fluid flow through the vessel.

A device according to the invention may also have spaces between the wires that are greater in the central portion of the device than at the ends of the device, as illustrated, for example, in Figures 9-15 and 17-20.

A device according to the invention may be constructed to any suitable size or in any suitable manner to accommodate a particular vessel, including veins and arteries (e.g., the abdominal aorta, aortic arch, the ascending aorta, the descending aorta, an iliac artery, or a renal artery). For example, the device may be used in wall apposition of a thoracic and/or abdominal endoluminal grafts, which means it expands to position at least a portion of a stent graft snugly (without a sheath) against the artery wall.

A device may be introduced into a vessel using either a biaxial (without a sheath) or triaxial (with a sheath) catheter, which is typically inserted over a guide wire. Optionally, the device includes one or more radio opaque markers that assist an operator in locating the device once in a vessel, although a device according to the invention can generally be seen using fluoroscopy without the need for radio opaque markers.

When dilated, a device according to the invention does not occlude or substantially hinder the flow of fluid through a vessel or into a side vessel because the fluids flow through the spaces (or openings) between the wires. In a pressure monitoring test using water as the fluid and a plastic tube to simulate the aorta the pressure drop within a vessel and downstream of a dilated device as generally shown in Figures 13-20 was measured as less than 1%. This test measured the flow lengthwise through the device, wherein the water had to flow through both the proximal end and distal end of the device. Thus, the water had to flow through the smallest openings in the device, which in the embodiment tested were located at the distal end and the proximal end. It is therefore believed that flow into a side vessel, wherein fluid would flow through the smaller openings in the distal end of the device and then through larger openings in the body portion of the device and into the side vessel, would be less hindered than flow lengthwise through the device. Accordingly, the pressure drop due to the dilation of a device according to the invention, either measured downstream of the proximal end of the device or measured in a side vessel covered by the device (such as when the device is in the aorta and covers one or both renal arteries), would be less than 70%, less than 60%, less than 50%, less than 40%, less than 30%, less than 20%, less than 10%, less than 5%, less than 2%, and/or less than 1%.

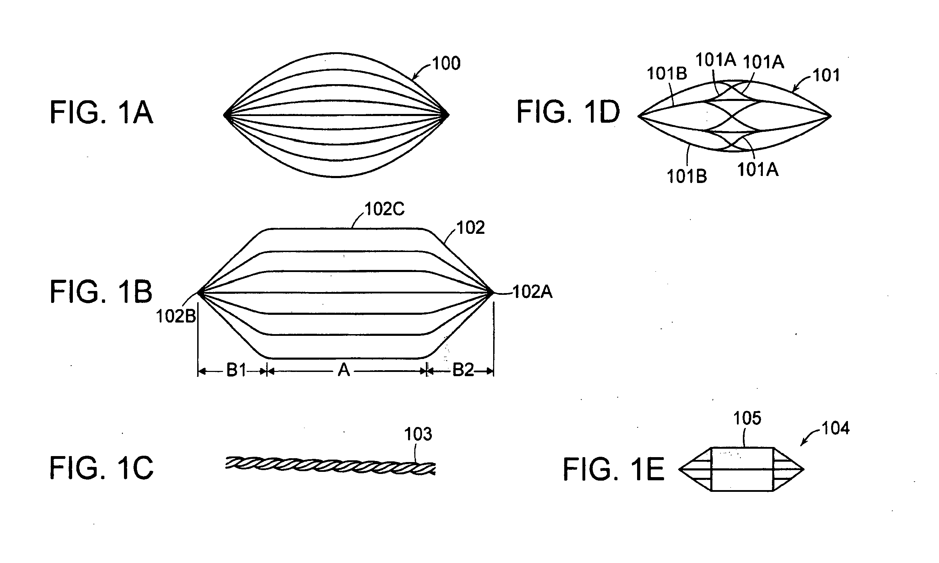

Reference will now be made to preferred embodiments of the invention, examples of which are illustrated in the accompanying drawings, wherein the purpose is to describe certain examples of the invention and not to limit the scope of the claims. Figures 1A-E show examples of spiraled devices according to various aspects of the invention. These devices are preferably dilated and collapsed by winding (to contract) and unwinding (to dilate) a plurality of wires that are preferably formed in a spiraled pattern. Device 100 shown in Figure 1A is a generally oval-shaped dilation device in a dilated position. Device 102 shown in Figure 1B is a dilation device with a substantially-linear section A of wires in the middle of device 102, while the wires in end sections B1 and B2 are at an angle so that they converge at approximately the same point at each respective end 102A, 102B on either side of device 102. In this way, section A of dilation device 102 may exert more even pressure against a vessel and/or structure within a vessel. In this example, the substantially-straight section A is approximately 3 cm in length, while each of the end sections B1 and B2 is approximately 1 cm in length. However, the device may be of any suitable size or shape and be constructed in any manner.

Device 101 shown in Figure 1D, is spiraled, shown in a dilated position and includes support members 101A between wires 101B. Support members 101A provide additional strength to device 101.

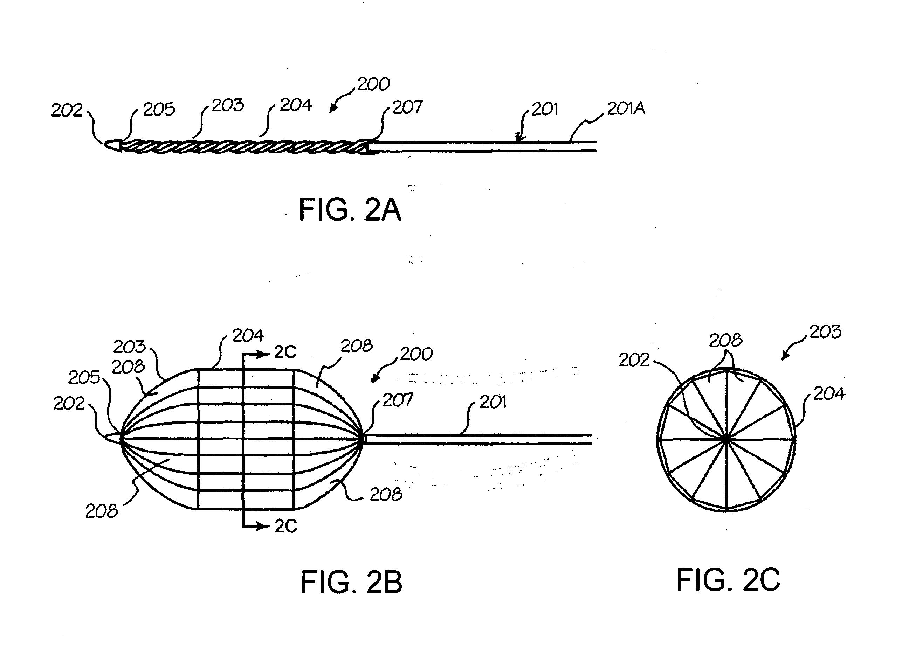

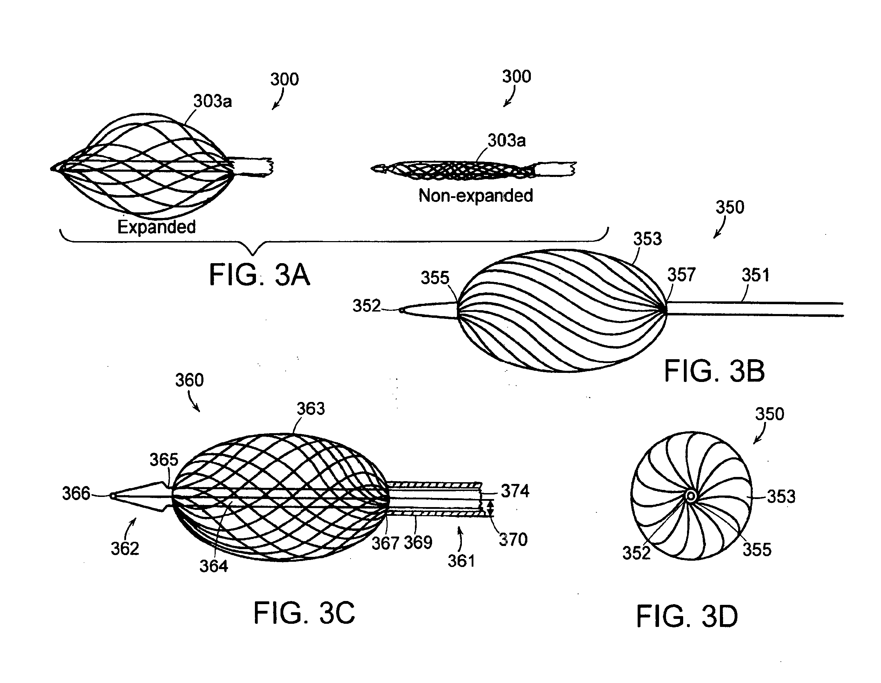

Device 103 shown in Figure 1C is an exaggerated view of wires in a spiraled dilation device such as device 100 when the wires are in a spiraled position. In this position, the diameter of the dilation device is reduced, allowing for insertion into a blood vessel. Unspiraling the wires causes the device to dilate, as shown in Figures 1A-1C. Other embodiments of a spiraled dilation device will be discussed further with regard to Figures 2A-C and Figures 3A-D.

Any device according to the invention may utilize a lining, such as lining 105 shown in Figure 1E. A lining such as lining 105 may be positioned on part of the exterior surface and/or interior surface of a device, such as 104. The use of a lining (a) provides a more even surface (depending upon the nature of the device with which it is used) for exerting pressure during the dilation process, and/or (b) helps to prevent the wires of the device from becoming entangled with exposed wires on a stent or stent graft.

Lining 105 is preferably made from a permeable material, which would be important if the lining is positioned such that it could occlude or seriously hinder blood flow. However, impermeable materials may used if the lining is not positioned where it could seriously hinder blood flow. For example, in device 104, even if an impermeable material is used for the lining, blood will still flow through the gaps between the wires at each end of the device. So as long as device 104 is not positioned so that it blocks a side vessel, or an impermeable membrane on device 104 is not positioned so that it blocks a side vessel, an impermeable material could be used. Any suitable material may be used for liner 105 and examples of preferable lining materials include, but are not limited to, polyurethane, PTFE (polytetrafluoroethylene), nylon, or any material used in carotid embolic protection devices.

Figures 2A-C show an assembly 200 according to an embodiment of the invention. Figure 2A shows spiraled dilation device 203 in a first position for insertion into a blood vessel. Assembly 200 also includes a biaxial catheter 201 (biaxial catheter does not include a catheter sheath) with a distal tip 202. Catheter 201 (and any catheter used with a device as described or claimed herein) may be made of any material suitable for insertion into a vessel and may be sized for a particular vessel. Catheter 201 has an outer tube 201 A, a central tube (not shown) running the length of the catheter. The central tube includes a wire port (not shown) in distal tip 202 and a lumen (not shown) for receiving a guide wire. Catheter 201 is inserted into a vessel by being guided over a guide wire going through the wire port and through the lumen in the central tube. Catheter 201 may be triaxial, in which case there would be a catheter sheath over device 203 (as described below).

Dilation device 203 is affixed to catheter 201 at point 205 and also at point 207. As shown in Figure 2A, dilation device 203 is spiraled around the central tube in catheter 201 in a first position. In this position, catheter 201 and dilation device 203 are positioned to be inserted into the vessel. Dilation device 203 may optionally include a lining 204, which may be one of the same types of linings as discussed above.

Figure 2B shows device 203 in an expanded position. Dilation device 203 is expanded by exerting a twisting motion on either outer tube 201 A (preferred) or on the central tube, while keeping the other of the two tubes relatively still so that device 203 can expand. Because dilation device 203 is affixed at point 205 to the central tube and at point 207 to outer tube 201 A, a twisting motion applied to outer tube 201 A, while keeping the central tube relatively still (at least with as little motion as necessary to allow device 203 to expand), will unspiral and dilate device 203. Optionally, the twisting motion will be applied to the central lumen.

Figure 2C shows a cross-sectional view taken along line A-A when dilation device 203 is in an expanded position. As can be seen, lining 204 provides for a more substantially uniform surface than would the wire mesh of dilation device 203 alone. Gaps 208 between the wires of dilation device 203 allow fluid to flow through device 203.

Figures 3A-3D show additional views of an assembly including a spiraled dilation device according to one embodiment of the invention. Figure 3A shows a spiral mesh structure rather than the straighter, cage-like structure of Figures 2A-C. The spiral mesh shown in Figures 3A-3D has a greater wire density when expanded than the structure shown in Figures 2A-C. The wire density (i.e., the number of wires in a given area) used in a dilation device may be varied for different applications. In general, the denser the wire mesh when a device is dilated, the more surface area available to press against a blood vessel and/or structure within the blood vessel.

Figure 3A shows dilation device 303a in an expanded position and in a non-expanded position. Figure 3B shows an assembly 350 including an expanded spiral mesh device 353 and catheter 351. Device 353 is affixed to catheter 351 at affixation points 355 and 357, catheter 351 also has a distal tip 352.

Figure 3C shows a partial, sectional side view of an assembly 360 with a dilation device 363 having a wire mesh structure, and a catheter 361 with a distal tip 362. While not necessary, a tapered front end on distal tip 362 allows for easier insertion into a vessel. At the end of distal tip 362 is a wire port 306, which leads to a lumen, for insertion of catheter 361 over a guide wire 370. The proximal end of distal tip 362 may have a reverse taper towards affixation point 365. Affixation point 365 is the point at which the distal end of dilation device 363 connects to central tube 364. Affixation point 367 is the point at which the proximal end of dilation device 303 connects to outer tube 369, which is positioned coaxially around central tube 364. Dilation device 363 is expanded by twisting outer tube 369 (or alternatively by twisting central tube 364).

Figure 3D shows a front view of device 350, which was previously described.

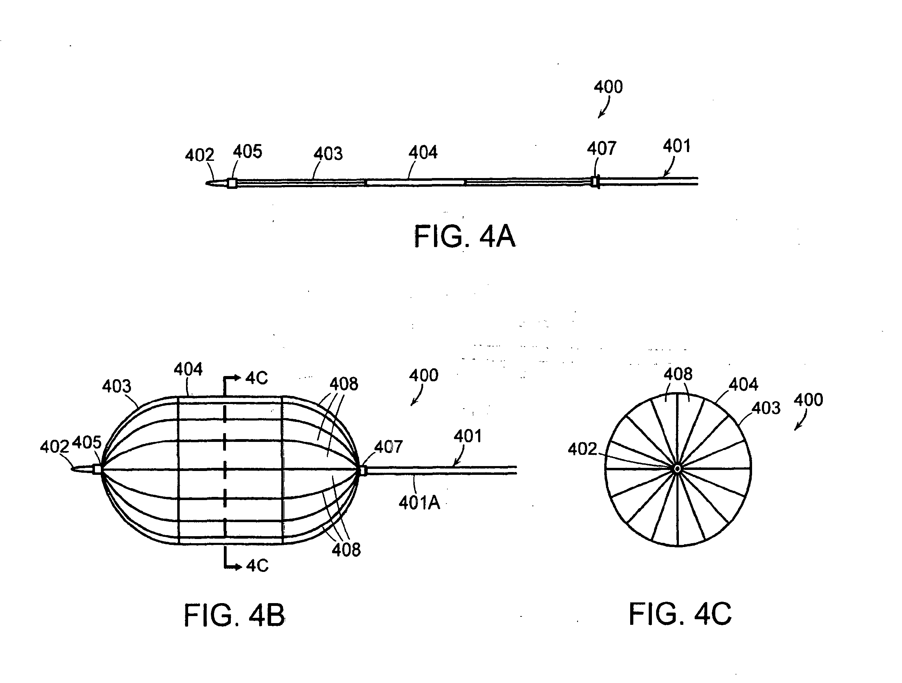

Figures 4A-C show an assembly 400 having a non-spiraled, expansive dilation device according to one embodiment of the invention. Figure 4A shows a non-spiraled, dilation device 403 in a first position for insertion into a blood vessel. Assembly 400 also includes a catheter 401 with a distal tip 402. Catheter 401 may be any device having a central lumen and being capable of insertion into a blood vessel over a guide wire. Catheter 401 has a central tube (not shown) with a wire port (not shown) in distal tip 402 that communicates with a lumen in the central tube. Catheter 401 is inserted into a blood vessel over a guide wire going through the wire port and into the lumen.

Dilation device 403 is affixed to catheter 401 at point 405 and also at point 407. As shown in Figure 4A, dilation device 403 is not spiraled. That is, each wire of dilation device 403 is substantially parallel to the other wires and runs in a substantially straight line from affixation point 405 (on a central tube, which is not shown) to affixation point 407 on outer tube 401 A. In this first position shown in Figure 4A, the catheter 401 and dilation device 403 are insertable into a vessel. Dilation device 403 may optionally include a lining 404 as discussed above with reference to Figure 1, which in this embodiment is on the inside surface of dilation device 403.

Figure 4B shows device 400 in an expanded position. Dilation device 403 is expanded by exerting linear pressure via catheter 401 (e.g., a push-pull motion). Because dilation device 403 is affixed at points 405 and 407, a linear motion applied to one tube of catheter 401 (such as by pulling the central tube or pushing outer tube 401 A) will expand device 403. As can be seen in Figure 4B, the use of optional lining 404 creates a substantially uniform surface for dilating blood vessels and structures.

Figures 4C shows a sectional view taken along line A-A when dilation device 403 is in the expanded position. Gaps 408 between the wires of dilation device 403 allow fluid to flow through device 403.

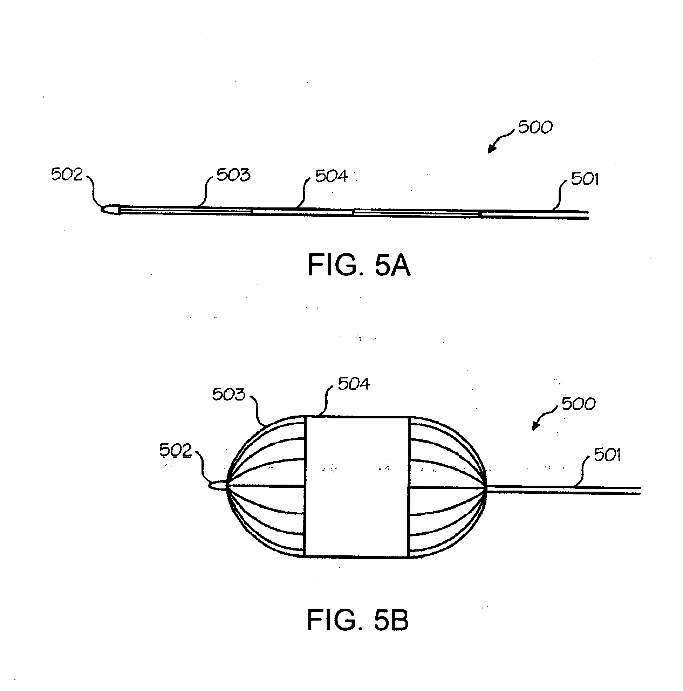

Figure 5 shows assembly 500 including a non-spiraled, expansive dilation device 503 according to one embodiment of the invention. Assembly 500 also includes a catheter 501 having a distal tip 502. Assembly 500 has the same structure as assembly 400 except that liner 504 is placed on the outside of dilation device 503.

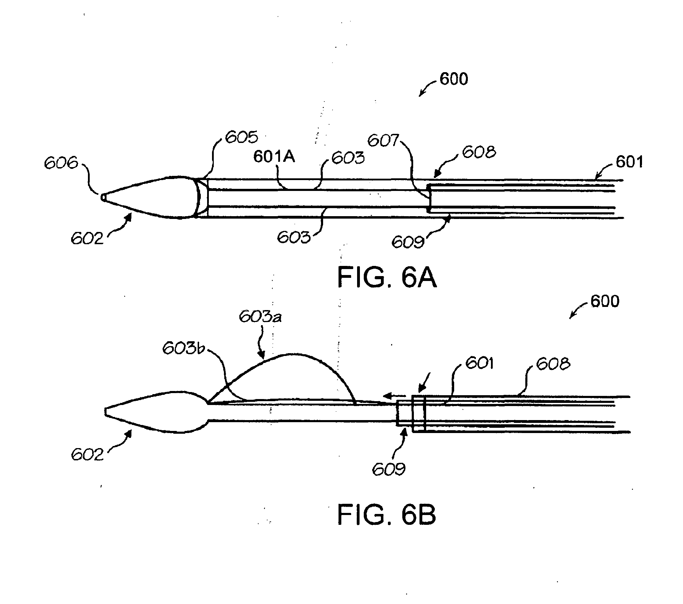

Figures 6A-B show an assembly 600 for a non-spiraled, dilation device according to an embodiment of the invention. Triaxial catheter 601 includes a central tube 601 A, an outer tube 609 and a catheter sheath 608. Wire port 606 may be constructed to fit over any size guide wire (e.g., port 606 may be a 0.1mm (.038" diameter) wire port). Affixation point 605 is where the distal end of dilation device 603 attaches to central tube 601 A. Outer tube 609 is positioned coaxially around central tube 601 A and the proximal end of dilation device 603 attaches to outer tube 609 at affixation point 607. Catheter sheath 608 is positioned coaxially around outer tube 609 and can be moved towards tip 602 to cover device 603 or away from tip 602 to expose tip 603.

Catheter sheath 608 may include radiopaque markers to indicate when device 603 has cleared the treatment zone.

Figure 6B shows dilation device 603 in two positions. In position 603a, dilation device 603 is expanded. The expansion is accomplished by pushing or otherwise moving (such as by using a screw mechanism) outer tube 609 forward (preferred) while keeping central tube 601 A relatively stationary or central tube 601 A backward while keeping outer tube 609 relatively stationary. In this manner the proximal end of dilation device 603 and the proximal end of dilation device 603 are moved towards each other and the wires of dilation device 603 expand outward. In position 603b, the wires of dilation device 603 remain at essentially their smallest diameter and close to central tube 601 A. If device 603 had been expanded, it is moved to the position shown in position 603b by increasing the distance between the distal end and proximal end of device 603 by either pulling outer tube 609 back or pushing central tube 601 A forward.

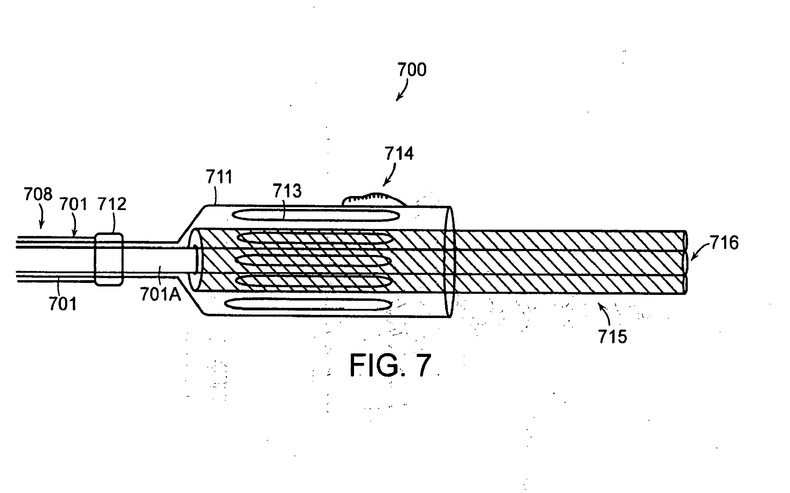

Figure 7 shows a control mechanism 700 for a dilation device according to one embodiment of the invention. Control mechanism 700 is the hand-held portion of a dilation assembly (which in this embodiment is a catheter that includes the controls and the device) and may be used with both spiraled and non-spiraled dilation devices. In the case of a non-spiraled, dilation device, handle 711 is attached to catheter sheath 708 through hemostatic valve 712. For both spiraled and non-spiraled dilation devices, central tube 701 A of catheter 701 runs through handle 711 and has a wire port 716 at its distal end that communicates with a lumen. As shown in Figure 7, handle 711 is a nut-type handle that is either fused to an outer sheath and may be twisted (for a spiraled dilation device) or pushed/pulled (for a non-spiraled, expansive dilation device) to engage or disengage a dilation device. Handle 71 1 may include surface texturing 713 for easier grip. Handle 71 1 may also include a threaded, bolt-type fixation handle 715 that is fused to catheter 701. This allows for execution of a twisting motion for spiraled dilation devices. Handle 711 may also include a thumb-controlled quick release 714. Quick release 714 disengages handle 711 from the bolt-type fixation handle, allowing push/pull motions to be exerted on the handle and any attached sheaths and/or catheters (e.g., for engaging non-spiraled dilation devices).

Figure 8 shows an alternate device 800 according to the invention that is shown in a dilated position. Device 800 is comprised of wires 801 and includes a proximal end 802 retained by a retention member 803 and a distal end 804 retained by a retention member 805. As used herein, the distal end and the proximal end are the parts of the device that extend about 15 mm from each respective retention member. Device 800 has a body portion 807 positioned between ends 802 and 804 and spaces 806 are formed between wires 801 when device 800 is dilated as shown. Spaces 806 are preferably (but not necessarily) greater between wires 801 in body portion 807 than the spaces 806 between the wires 801 at end 802 or end 803 when device 800 is dilated. A band of wires 850 may be formed near the center of body portion 807 to add greater radial strength, and the spaces between the wires 801 in such a band are typically smaller than the spaces between the wires 801 in other parts of body portion 807.

Figure 9 shows a device 900 according to the invention that is in the dilated position and comprises a plurality of wires 901. In this embodiment each wire 901 is parallel to the other wires 901 (in this context "parallel" means substantially parallel). Each of the wires 901 is also parallel to the vessel flow path when device 900 is inserted into a vessel (again, in this context, "parallel" means substantially parallel). Device 900 as shown is formed by slitting a tube and has unslitted ends 902, 904 a proximal end 906 and a distal end 908. Device 900 has a body portion 910 between proximal end 906 and distal end 908. As shown, wires 901 are formed in three-wire groups with distances 912 between the groups and distances 914 between wires in each group. Distances 912 are greater than distances 914 and each of the respective distances 912 and 914 are greater in body portion 910 than they are at either proximal end 906 or distal end 908.

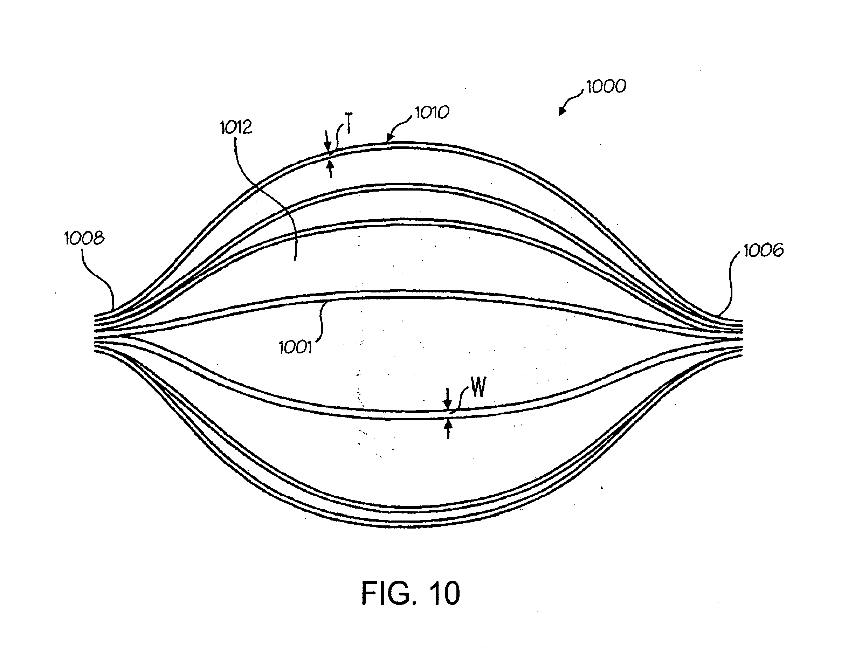

Figure 10 shows a device 1000 that is in a dilated position. Device 1000 comprises a plurality of wires 1001 and is preferably formed by slitting a tube and leaving the ends of the tube (not shown in this Figure) unslit. In this embodiment each of the wires 1001 is parallel (in this context "parallel" means substantially parallel) to the other wires 1001 and each of the wires 1001 is also parallel (again, in this context, "parallel" means substantially parallel) to the vessel flow path when device 1000 is positioned in a vessel. Each wire 1001 is preferably a strut having a generally rectangular cross section and preferably having a width greater than its thickness. The width could be any suitable width but is preferably between 0.5mm (.020") and 1.27mm (.050") and the thickness could be any suitable thickness but is preferably between 0.2mm (.008") and 4.6mm (.018"). Device 1000 has a proximal end 1006, a distal end and 1008 and a body portion 1010. There is a distance 1012 between wires 1001 and in this embodiment the distance 1012 is greater in body portion 1010 than in either proximal end 1006 or distal end 1008.

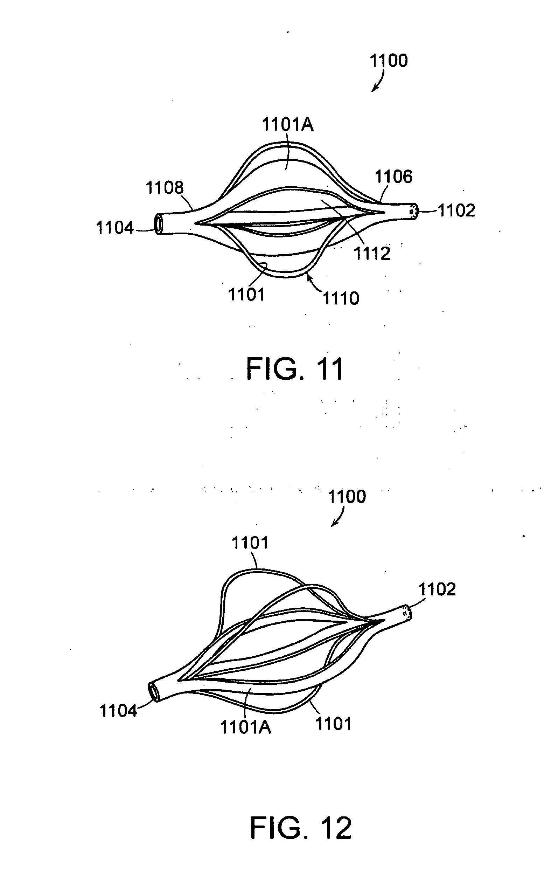

Figures 11 and 12 show a device 1100 according to the invention that is in a dilated position and that comprises a plurality of wires 1101. In this embodiment each wire 1101 is parallel to the other wires 1101 (in this context "parallel" means substantially parallel). Each of the wires 1101 are also parallel to the vessel flow path when device 1100 is inserted into a vessel (again, in this context, "parallel" means substantially parallel). Device 1100 as shown is formed by slitting a tube and has unslitted ends 1102 and 1104 (shown in Figure 12) that are connected, respectively, to proximal end 1106 and distal end 1108. Device 1100 has a body portion 1110 between proximal end 1106 and distal end 1108. Device 1100 has two types of wires, wires 1101 and 1101 A. As shown wires 1101 are slender, having a preferred width of between about 0.2mm (.008") and 0.4mm (.014") whereas wires 1101A are wider and have a width of between about 0.5mm (.020") and 0.6mm (.025"). Wires 1 101 also extend further from the center of body portion 1110 than do wires 1101A. In this embodiment wires 1101 and 1101A function together to apply even pressure to a substantial area of a vessel and/or apply even pressure to a substantial area of a structure to be positioned within a vessel.

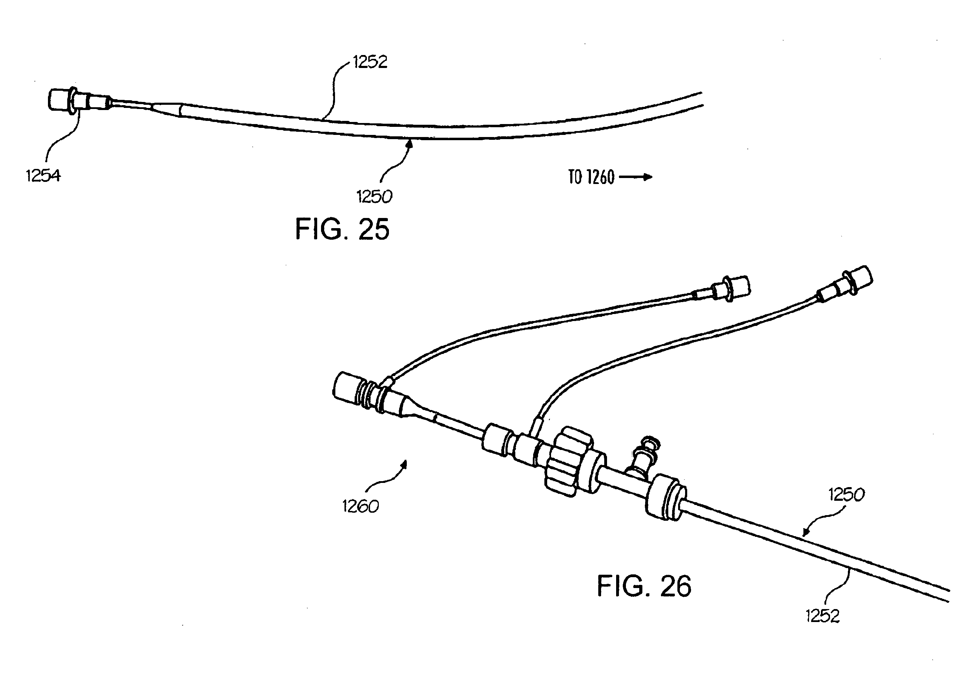

Figure 13 shows a device 1200 according to the invention that is mounted on a catheter 1250. Catheter 1250 is of a triaxial design generally known in the art and includes a catheter sheath 1252, a proximal end 1260 (best seen in Figure 26), which is outside of the patient's body during a procedure and is juxtaposed the operator when catheter 1250 is in use, and a distal end 1254 that is inserted into the body.

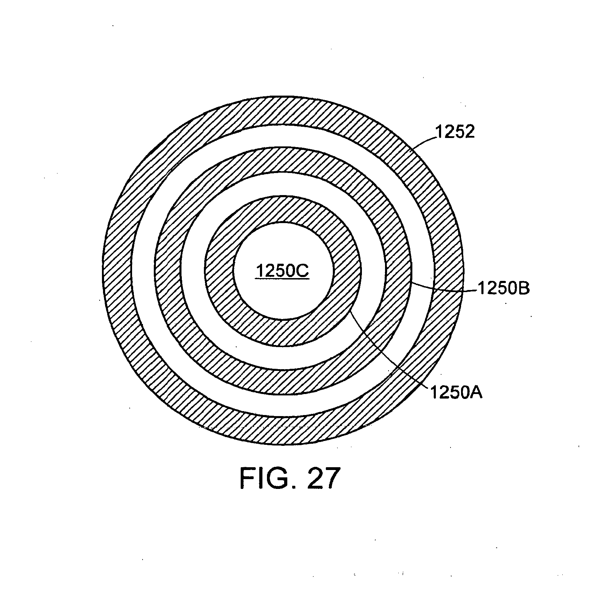

Figure 27 shows a cross-sectional view of catheter 1250 taken through line C-C of Figure 13. Catheter 1250 includes, but is not limited to, three preferably coaxial tubes; central tube 1250A, outer tube 1250B and catheter sheath 1252. In this embodiment, central tube 1250A extends essentially the entire length of catheter 1250 and has a central lumen 1250C for receiving a guide wire (not shown). Central tube 1250A extends through device 1200 and is attached to device 1200 at end 1204. Outer tube 1250B is positioned over central tube 1250A and extends to end 1202 of device 1200 where it is connected to end 1202. Catheter sheath 1252 has a length sufficient to cover device 1200.

In operation the assembly including device 1200 and catheter 1250 is placed into a vessel with catheter sheath 1252 at least partially covering device 1200 to help retain it in its collapsed position and to allow for ease in directing the catheter and device through the vessel.

Once device 1200 is properly positioned in a vessel, catheter sheath 1252 is pulled back to expose device 1200. Device 1200 can then be dilated by either pushing outer tube 1250B, pulling central tube 1250A or by simultaneously pushing outer tube 1250B and pulling central tube 1250A. As previously explained, the tube that is not being pushed or pulled must remain stable enough so that the distance between retention ends 1202 and 1204 decreases and device 1200 expands.

If a device according to the invention were being used to position a structure in the vessel, the structure (such as a stent or stent graft) could be mounted on the device in a typical manner known to those in the art so that as the device dilates the structure is dilated.

Utilizing catheter 1250 (or any suitable biaxial or triaxial catheter) a device, such as device 1200 or 1300, is dilated by moving the distal and proximal ends of the device towards each other. The device is contracted and collapsed by releasing the force pushing the two ends together and/or by moving the two ends apart.

Alternatively, any device according to the invention may be preformed in a dilated position and compressed into a collapsed position when covered by catheter sheath 1252. When catheter sheath 1252 is removed the preformed device would immediately expand to its dilated position and then could be contracted or further dilated by an operator utilizing the catheter in one of the manners described.

In Figure 13, device 1200 is shown in its dilated position and it comprises a plurality of wires 1201 that are formed in a criss-cross pattern. Device 1200 has retention ends 1202 and 1204 that may be formed as part of catheter 1250, a proximal end 1206, a distal end 1208 and a body portion 1210. Spaces 1212 are formed between wires 1201 and can be of any suitable size, e.g., between about 1 mm² and about 400 mm². As shown, spaces 1212 are larger in body portion 1210 that in either proximal end 1206 or distal end 1208.

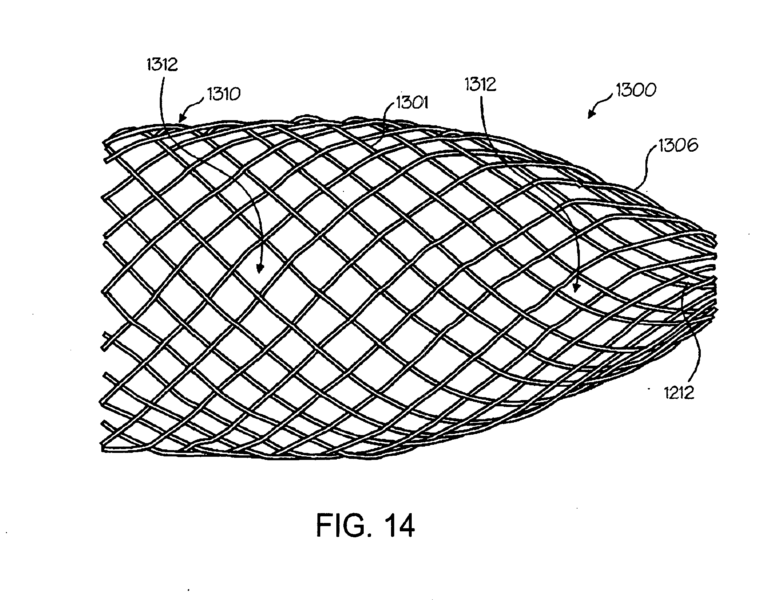

Figure 14 is a close-up, partial side view of an alternate device 1300 showing proximal end 1306 and part of body portion 1310. As can be seen spaces 1312 between wires 1301 are smaller at proximal end 1306 than at body portion 1310.

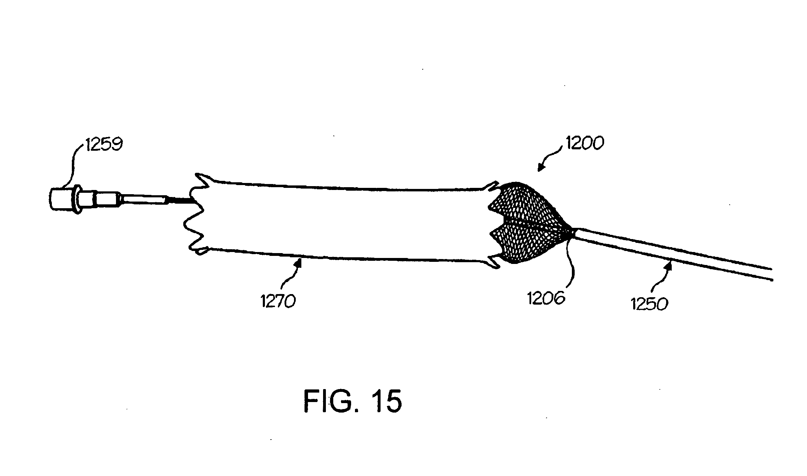

Figure 15 generally illustrates how device 1200 can be utilized to dilate a stent graft 1270, which is shown in a dilated position. Device 1200 is positioned inside of the portion of the stent graft that will be compressed against a vessel wall to anchor the stent graft in place. As the device is expanded it presses the stent graft against the vessel wall.

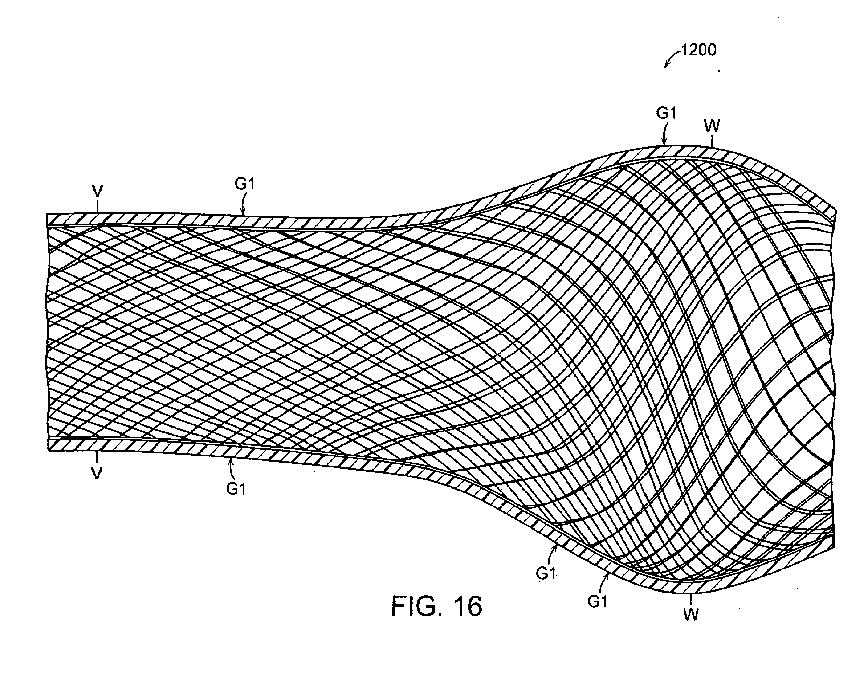

Figure 16 shows device 1300 dilated in a plastic model G1 to simulate device 1200 conforming to a diameter disparity ratio of approximately 1.8:1 in a vessel. Device 1300 is pressed against the entire interior wall of model G1 from at least position V to a position past position W.

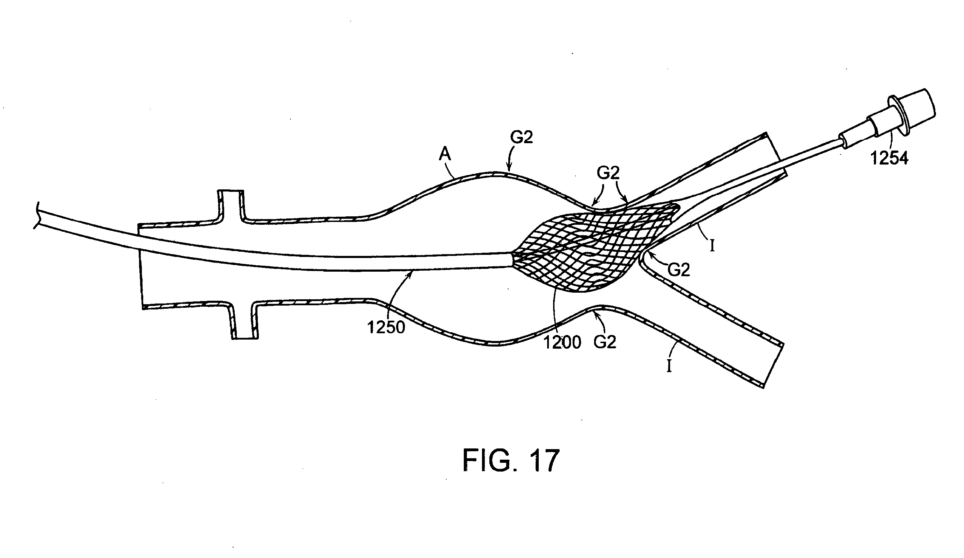

Figure 17 shows device 1200 and catheter 1270 in a plastic model G2 that simulates the aorta A and the iliac arteries I. In this Figure device 1200 is simultaneously positioned in the aorta and an iliac artery and is conforming to a multi-vessel diameter disparity ratio of about 2.0:1.

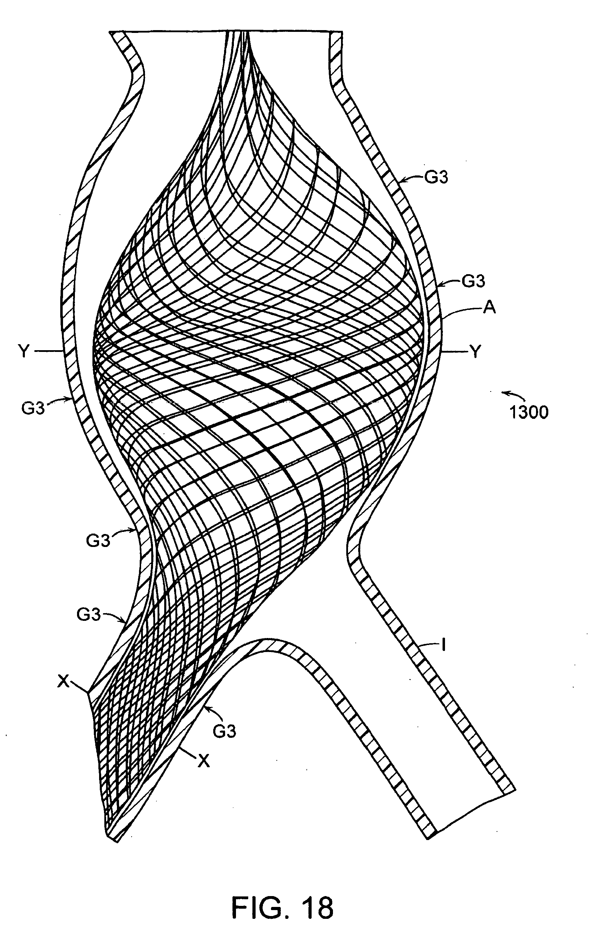

Figure 18 shows a device 1300 in accordance with the invention that is dilated in a plastic model G3 to simulate device 1300 being dilated simultaneously in the aorta A and an iliac artery I. In this Figure device 1300 is conforming to a multi-vessel diameter disparity ratio of about 3.4:1. Device 1300 is pressed against the entire interior wall of model G3 (except for one of the simulated iliac arteries I that does not include device 1300) from at least position X to a position past position Y.

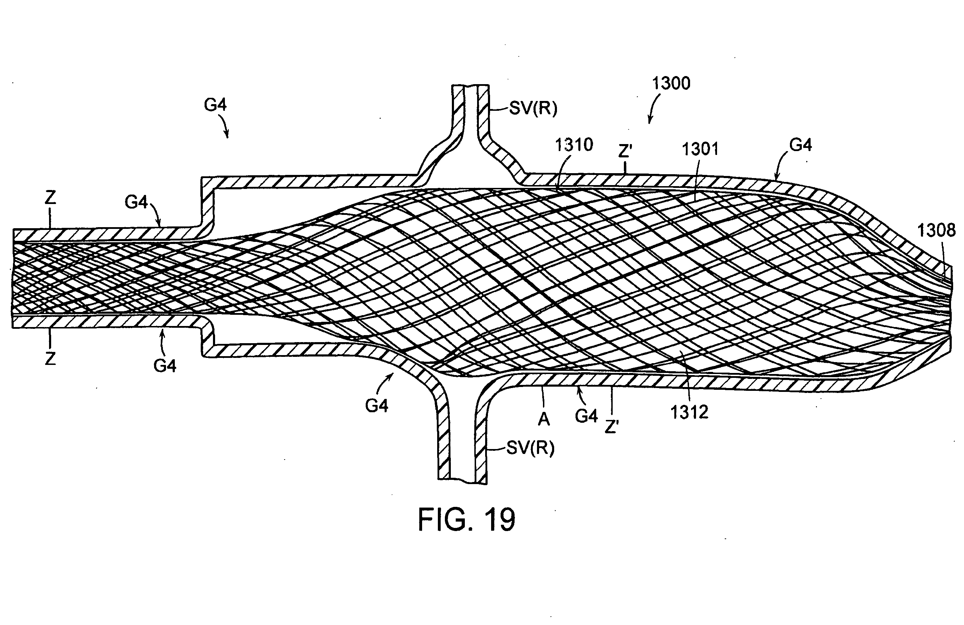

Figure 19 shows device 1300 with wires 1301, proximal end 1308 and spaces 1312 between wires 1301. Device 1300 is dilated in a plastic model G4 to simulate device 1300 being dilated in aorta A and covering side vessels SV(R) that simulate the renal arteries. As can be seen, fluid would flow through the spaces 1312 at proximal end 1308, through the aorta and into the side vessels through spaces 1312 in body portion 1310. In this Figure, device 1300 is also conforming to a vessel diameter disparity ratio of about 2.0:1. Device 1300 is pressed against the entire interior wall of model G4 (except for the simulated renal arteries SV (R) shown as side vessels that are covered by device 1300) from at least position Z to a position past position Z'.

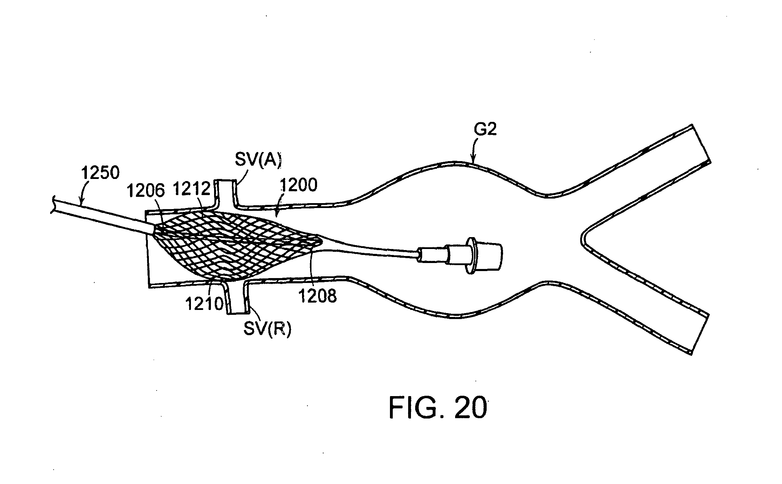

Figure 20 shows device 1200 and catheter 1250 positioned in a plastic model G2 to simulate device 1200 being positioned and dilated in the aorta and covering side branches, such as the renal arteries SV(R). The spaces 1212 between the wires 1201 in device 1200 allow fluid to flow through the aorta and into the side vessels when device 1200 is dilated.

Figure 21 shows the device of Figure 13 in its collapsed position and having a kink radius of 13.5 mm.

Figure 22 shows the device of Figure 13 in its fully dilated position and having a kink radius of 16 mm.

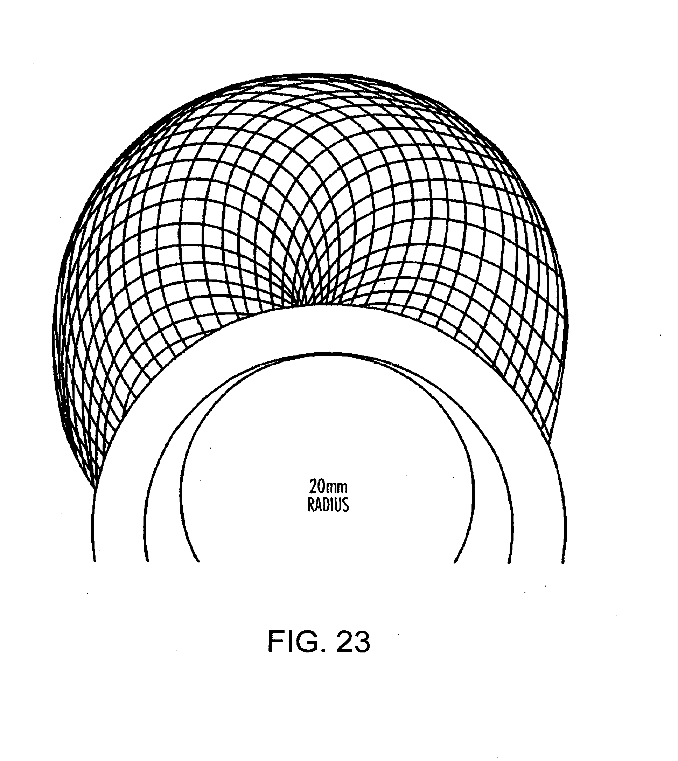

Figure 23 shows the device of Figure 13 in its fully dilated position and having a kink radius of 20 mm.

A device according to the present invention thus may have a kink radius of 13.5 mm or greater before being dilated. This includes one or more of a kink radii of 14.0 mm, 15.0 mm, 16.0 mm, 17.0 mm, 18.0 mm, 19.0 mm, 20.0 mm and greater. Further, a device according to the present invention may, when fully dilated, have a kink radius of 16.0 mm or greater. This includes one or more of the kink radii of 17.0 mm, 18.0 mm, 19.0 mm, 200 mm, 21.0 mm, 22.0 mm, 23.0 mm, 24.0 mm, 25.0 mm and greater.

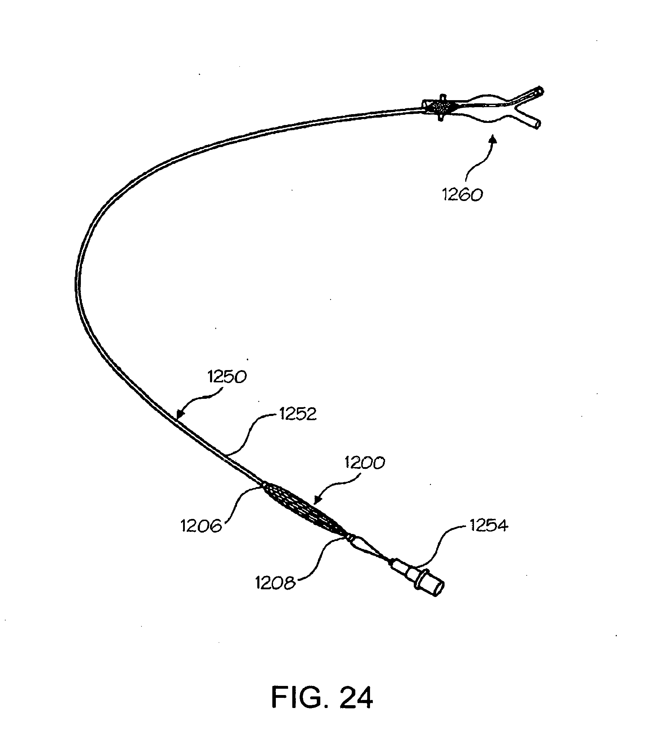

Figure 24 shows the catheter 1250 of Figure 13 that includes device 1200. Catheter 1250 has a proximal end 1254 that is inserted into a vessel during use, and a distal end 1260 that remains outside of the vessel and is used by an operator to position, release and dilate device 1200.

Figure 25 shows proximal end 1254 of catheter 1250.

Figure 26 shows distal end 1260 of catheter 1250. Other embodiments of the invention will be apparent to those skilled in the art from consideration of the specification and embodiments disclosed herein. Thus, the specification and examples are exemplary only, with the true scope of the invention set forth in the following claims and legal equivalents thereof.

## Claims

1. An assembly comprising a dilation device (1200) for dilating a vessel or a structure positioned within a vessel, the device (1200) comprising a plurality of wires (1201) comprised of nitinol and having a generally circular cross section and a diameter of between 0.203 mm (0.008 inches) and 0.457 mm (0.018 inches), wherein the plurality of wires (1201) form a body portion (1210) having a proximal end (1206) and a distal end (1208), wherein the plurality of wires (1201) form a criss-cross pattern and are spaced apart so that at least some of the spaces between the wires has an area of between 1 mm² and 400 mm² when the device (1200) is dilated to allow for passage of fluid through the device (1200), the device (1200) being sufficiently compliant to conform to at least one diameter disparity ratio of between about 1.2:1 and about 3.4:1 when dilated, wherein the diameter disparity ratio is the disparity of the diameter of the vessel, the assembly further comprising a catheter (1250) for engaging the device (1200), wherein a first retention end (1202) of the catheter (1250) engages the proximal end (1206) of the body portion (1210) and a second retention end (1204) of the catheter (1250) engages the distal end (1208) of the body portion (1210).

2. The assembly of claim 1 wherein the device (1200) is sufficiently compliant so that the device (1200) can conform to a multi-vessel diameter disparity ratio of between about 1.2:1 and about 3.4:1, wherein the multi-vessel diameter disparity ratio is the disparity of the diameters of two vessels.

3. The assembly of claim 1 wherein the device (1200) has a kink radius of greater than or equal to about 13.5 mm prior to being dilated.

4. The assembly of claim 1 wherein the device (1200) has a kink radius of between about 16.0 mm and about 20.0 mm after being fully dilated.

5. The assembly of claim 1 wherein spaces (1212) formed in the body portion (1210) are larger than spaces (1212) formed at the distal end (1208) or the proximal end (1206).

6. The assembly of claim 1 wherein the catheter (1250) includes a central tube (1250A) extending through the device (1200) and engaging the second retention end (1204) of the body portion (1210), an outer tube (1250B) positioned over the central tube (1250A) and engaging the first retention end (1202) of the body portion (1210), and a catheter sheath (1252) that at least partially encloses the device (1200) during insertion of the device (1200).

7. The assembly of claim 6 wherein the device (1200) is preshaped to automatically expand when released from the catheter sheath (1252).

8. The assembly of claim 1 wherein the device (1200) has a fully collapsed diameter of less than 4 mm (12 french) and a fully expanded diameter of between about 30 mm - about 35 mm.

9. The assembly of claim 1 wherein the device (1200) has a fully collapsed diameter of less than about 5 mm (15 french) and a fully expanded diameter of between about 50 mm and about 55 mm.

10. The assembly of claim 1 further comprising a permeable membrane on at least part of the device.

## Patentansprüche

1. Anordnung, umfassend eine Dilatationsvorrichtung (1200) zum Dilatieren eines Gefäßes oder einer Struktur, die innerhalb eines Gefäßes positioniert wird, wobei die Vorrichtung (1200) eine Vielzahl Drähte (1201) aufweist, die aus Nitinol bestehen und im Allgemeinen einen kreisförmigen Querschnitt und einen Durchmesser von zwischen 0,203 mm (0,008 Inch) und 0,457 mm (0,018 Inch) aufweisen, wobei die Vielzahl Drähte (1201) einen Körperabschnitt (1210) bilden, der ein proximales Ende (1206) und ein distales Ende (1208) aufweist, wobei die Vielzahl Drähte (1201) ein kreuzförmiges Muster bilden und derart voneinander beabstandet sind, dass mindestens einige der Abstände zwischen den Drähten eine Fläche von zwischen 1 mm² und 400 mm² aufweisen, wenn die Vorrichtung (1200) dilatiert ist, um ein Durchlaufen von Flüssigkeit durch die Vorrichtung (1200) zu ermöglichen, wobei die Vorrichtung (1200) ausreichend nachgiebig ist, um sich mindestens einem Durchmesserunterschieds-Verhältnis von zwischen ungefähr 1,2:1 und ungefähr 3,4:1 anzupassen, wenn sie dilatiert ist, wobei das Durchmesserunterschieds-Verhältnis der Unterschied von dem Durchmesser des Gefäßes ist, die Anordnung ferner einen Katheter (1250) für den Eingriff mit der Vorrichtung (1200) aufweist, wobei ein erstes Aufnahmeende (1202) von dem Katheter (1250) in das proximale Ende (1206) des Körperabschnitts (1210) eingreift und ein zweites Aufnahmeende (1204) des Katheters (1250) in das distale Ende (1208) des Körperabschnitts (1210) eingreift.

2. Anordnung nach Anspruch 1, wobei die Vorrichtung (1200) ausreichend nachgiebig ist, sodass die Vorrichtung (1200) sich einem Mehrgefäß-Durchmesserunterschieds-Verhältnis von zwischen ungefähr 1,2:1 und ungefähr 3,4:1 anpassen kann, wobei das Mehrgefäß-Durchmesserunterschieds-Verhältnis der Unterschied von dem Durchmesser von zwei Gefäßen ist.

3. Anordnung nach Anspruch 1, wobei die Vorrichtung (1200) einen Knickradius größer oder gleich von ungefähr 13,5 mm aufweist, bevor sie geweitet wird.

4. Anordnung nach Anspruch 1, wobei die Vorrichtung (1200) einen Knickradius zwischen ungefähr 16,0 mm und ungefähr 20,0 mm aufweist, nachdem sie vollständig geweitet ist.

5. Anordnung nach Anspruch 1, wobei Zwischenräume (1212), die im Körperabschnitt (1210) gebildet werden, größer sind als Zwischenräume (1212), die am distalen Ende (1208) oder dem proximalen Ende (1206) gebildet werden.

6. Anordnung nach Anspruch 1, wobei der Katheter (1250) ein zentrales Rohr (1250A), das sich durch die Vorrichtung (1200) erstreckt und in das zweite Aufnahmeende (1204) des Körperabschnitts (1210) eingreift, ein äußeres Rohr (1250B), das über dem zentralen Rohr (1250A) positioniert ist und in das erste Aufnahmeende (1202) des Körperabschnitts (1210) eingreift und eine Katheterhülle (1252) aufweist, welche die Vorrichtung (1200) während der Einführung der Vorrichtung (1200) mindestens teilweise umschließt.

7. Anordnung nach Anspruch 6, wobei die Vorrichtung (1200) vorgeformt ist, sich automatisch auszudehnen, wenn sie von der Katheterhülle (1252) freigesetzt wird.

8. Anordnung nach Anspruch 1, wobei die Vorrichtung (1200) einen Durchmesser im vollständig zusammengefalteten Zustand von weniger als 4 mm (12 French) und einen Durchmesser im vollständig geweiteten Zustand von zwischen ungefähr 30 mm-ungefähr 35 mm aufweist.

9. Anordnung nach Anspruch 1, wobei die Vorrichtung (1200) einen Durchmesser im vollständig zusammengefalteten Zustand von weniger als ungefähr 5 mm (15 French) und einen Durchmesser im vollständig geweiteten Zustand von zwischen ungefähr 50 mm und ungefähr 55 mm aufweist.

10. Anordnung nach Anspruch 1, ferner umfassend eine durchlässige Membran an mindestens einem Teilbereich der Vorrichtung.

## Revendications

1. Ensemble comprenant un dispositif de dilatation (1200) pour dilater un vaisseau ou une structure positionnée à l'intérieur d'un vaisseau, le dispositif (1200) comprenant une pluralité de câbles (1201) composés de nitinol et ayant une section transversale globalement circulaire et un diamètre entre 0,203 mm (0,008 pouce) et 0,457 mm (0,018 pouce), dans lequel la pluralité de câbles (1201) forme une partie formant corps (1210) ayant une extrémité proximale (1206) et une extrémité distale (1208), dans lequel les plusieurs câbles (1201) forment un motif en croix et sont espacés de sorte qu'au moins certains des espaces entre les câbles ont une surface entre 1 mm² et 400 mm² lorsque le dispositif (1200) est dilaté pour permettre le passage de fluide à travers le dispositif (1200), le dispositif (1200) étant suffisamment docile pour se conformer à au moins un rapport de disparité de diamètre entre environ 1,2:1 et environ 3,4:1 quand il est dilaté, dans lequel le rapport de disparité de diamètre est la disparité du diamètre du vaisseau, l'ensemble comprenant en outre un cathéter (1250) pour mettre en prise le dispositif (1200), dans lequel une première extrémité de retenue (1202) du cathéter (1250) met en prise l'extrémité proximale (1206) de la partie formant corps (1210) et une seconde extrémité de retenue (1204) du cathéter (1250) met en prise l'extrémité distale (1208) de la partie formant corps (1210).

2. Ensemble selon la revendication 1, dans lequel le dispositif (1200) est suffisamment docile de sorte que le dispositif (1200) peut se conformer à un rapport de disparité de diamètre multi-vaisseau entre environ 1,2:1 et environ 3,4:1, dans lequel le rapport de disparité de diamètre multi-vaisseau est la disparité des diamètres de deux vaisseaux.

3. Ensemble selon la revendication 1, dans lequel le dispositif (1200) a un rayon de courbure supérieur ou égal à environ 13,5 mm avant dilatation.

4. Ensemble selon la revendication 1, dans lequel le dispositif (1200) a un rayon de courbure entre environ 16,0 mm et environ 20,0 mm après dilatation totale.

5. Ensemble selon la revendication 1, dans lequel des espaces (1212) formés dans la partie formant corps (1210) sont plus grands que des espaces (1212) formés au niveau de l'extrémité distale (1208) ou de l'extrémité proximale (1206).

6. Ensemble selon la revendication 1, dans lequel le cathéter (1250) inclut un tube central (1250A) s'étendant à travers le dispositif (1200) et mettant en prise la seconde extrémité de retenue (1204) de la partie formant corps (1210), un tube extérieur (1250B) positionné sur le tube central (1250A) et mettant en prise la première extrémité de retenue (1202) de la partie formant corps (1210), et une gaine de cathéter (1252) qui englobe au moins partiellement le dispositif (1200) pendant l'insertion du dispositif (1200).

7. Ensemble selon la revendication 6, dans lequel le dispositif (1200) est préformé pour se dilater automatiquement quand il est libéré de la gaine de cathéter (1252).

8. Ensemble selon la revendication 1, dans lequel le dispositif (1200) a un diamètre totalement écrasé de moins de 4 mm (12 charrières) et un diamètre entièrement dilaté entre environ 30 mm à environ 35 mm.

9. Ensemble selon la revendication 1, dans lequel le dispositif (1200) a un diamètre totalement écrasé inférieur à environ 5 mm (1,5 charrière) et un diamètre entièrement dilaté entre environ 50 mm et environ 55 mm.

10. Ensemble selon la revendication 1, comprenant en outre une membrane perméable sur au moins une partie du dispositif.
